(19) Europäisches Patentamt
European Patent Office
Office européen des brevets

(11) EP 3 753 434 A1

(12) **EUROPEAN PATENT APPLICATION**
published in accordance with Art. 153(4) EPC

(43) Date of publication:
**23.12.2020 Bulletin 2020/52**

(21) Application number: **19754112.1**

(22) Date of filing: **12.02.2019**

(51) Int Cl.:
***A41G 3/00*** *(2006.01)* ***D06M 13/12*** *(2006.01)*
***D06M 13/152*** *(2006.01)* ***D06M 15/41*** *(2006.01)*
***D06M 15/423*** *(2006.01)*

(86) International application number:
**PCT/JP2019/004788**

(87) International publication number:
**WO 2019/159867 (22.08.2019 Gazette 2019/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.02.2018 JP 2018024956**

(71) Applicant: **Kao Corporation**
**Chuo-ku,**
**Tokyo 103-8210 (JP)**

(72) Inventor: **FURUKAWA, Junichi**
**Tokyo 131-8501 (JP)**

(74) Representative: **Hoffmann Eitle**
**Patent- und Rechtsanwälte PartmbB**
**Arabellastraße 30**
**81925 München (DE)**

(54) **HUMAN HAIR FIBER TREATMENT AGENT**

(57) A human hair fiber treatment agent for treating human hair fibers having free ends at both ends, comprising the following components (A) to (C) in the formulation thereof:
(A): formaldehyde or hydrate thereof;
(B): melamine derivative represented by the formula (1):

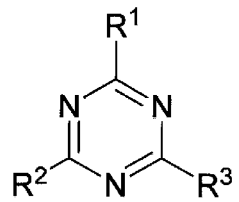

**(1)**

wherein $R^1$ to $R^3$ are the same or different and each represent a hydrogen atom, a hydroxymethylamino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms; and
(C): water.

EP 3 753 434 A1

## Description

Field of the Invention

The present invention relates to a human hair fiber treatment agent which semi-permanently or permanently changes the shape of human hair fibers as an industrial material.

Background of the Invention

Heretofore, synthetic hair fibers and human hair fibers have been widely known as fibers for hair implantation for making wigs.

Wigs using synthetic hair provide the following advantages: their high heat resistance easily allows straight hair to be curled or curled hair to be straightened using a heating tool such as a curling iron (hereinafter, referred to as "shape imparting properties"), and such a curled or straight shape formed using a heating tool is retained even under hot and humid conditions such as bathing or hair wash (hereinafter, referred to as "shape sustainability"). Furthermore, the wigs using synthetic hair are also excellent because such wigs are more rigid and have higher strength as compared with wigs using human hair. On the other hand, the wigs using synthetic hair have the following disadvantages: gloss is strong due to smooth surface, and the original purpose of wearing natural feeling of hair with a wig is difficult to achieve due to unnatural texture.

Wigs using human hair provide the advantages that the wigs have texture or gloss similar to those of self hair and produce natural appearance upon wearing. Most people who wear wigs do not want to be known to anyone as wearing wigs. Under such circumstances, it is largely advantageous to produce natural appearance on the wig.

For example, Patent Literature 1 discloses a fiber tress for hair prepared by mixing human hair with polyester fibers having specific physical properties, in order to overcome the disadvantage for poor shape sustainability of human hair while maintaining the excellent characteristics of human hair. Patent Literature 2 discloses a hair ornament product prepared by blending regenerated collagen fibers with human hair, in order to overcome the disadvantage for accumulation of frizz of human hair products, and also to supplement permanent wave performance which is a disadvantage of regenerated collagen fibers.

(Patent Literature 1) International Publication No. WO 2005/037000
(Patent Literature 2) JP-A-2007-177370

Summary of Invention

The present invention provides a human hair fiber treatment agent for treating human hair fibers having free ends at both ends, comprising the following components (A) to (C) in the formulation thereof:

(A): formaldehyde or hydrate thereof;
(B): melamine derivative represented by the formula (1):

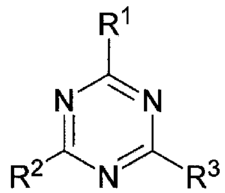

**(1)**

wherein $R^1$ to $R^3$ are the same or different and each represent a hydrogen atom, a hydroxymethylamino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms; and
(C): water.

The present invention further provides a method for treating human hair fibers, comprising the following step (i):

(i) the step of immersing human hair fibers having free ends at both ends in a human hair fiber treatment agent

described above under heating.

The present invention further provides a method for producing a wig, comprising the step of treating human hair fibers for wigs by the method for treating human hair fibers described above.

Detailed Description of the Invention

Human hair currently used in hair ornament products such as wigs undergoes many treatment steps including removal of cuticle by chemical treatment as well as bleaching and dyeing, in order to impart uniform shapes or colors thereto. Therefore, the human hair may be damaged during these steps (Patent Literature 1) and is known to present problems associated with durability (Patent Literature 2).

In recent years, an increasing number of people have given weight to appearance because of increasing needs for aesthetics. Thus, there has been a demand for wigs having higher durability.

However, in the techniques described in Patent Literatures 1 and 2, the disadvantages of unnatural appearance and poor feel are still not solved because human hair is mixed with synthetic hair. Thus, these techniques are insufficient for providing natural appearance.

Thus, the present invention relates to a human hair fiber treatment agent for producing human hair fibers for wigs which are excellent in shape sustainability, and durability while retaining the natural appearance of human hair, as well as to a method for treating human hair fibers.

The present inventors have found that the treatment of human hair fibers for hair implantation to wigs or a wig having implanted human hair fibers in hair with a composition containing specific aldehyde compound and specific phenol compound can not only impart shape sustainability to the human hair fibers but also improve the strength of the human hair fibers, thereby improving durability; and that as a result, the treated human hair fibers satisfy shape sustainability, natural gloss, good feel, and high durability simultaneously. Thus the present inventors have completed the invention.

According to the present invention, human hair fibers for wigs which are excellent in shape sustainability and durability while retaining the natural appearance and good feel of human hair can be produced.

In the present invention, the human hair fibers having free ends at both ends refer to human hair fibers which are head hair separated from the human head, and are used as an industrial material or a craft material such as hair for wigs, hair extensions, or hair implantation.

[Component (A): formaldehyde or hydrate thereof]

The component (A) is formaldehyde or hydrate thereof. Examples of the formaldehyde hydrate include formaldehyde monohydrate (methanediol). Among these, formaldehyde is preferable in view of imparting higher shape sustainability and durability to the treated human hair fibers.

The content of the component (A) in the human hair fiber treatment agent of the present invention is preferably 0.1% by mass or more, more preferably 1% by mass or more, further preferably 2.5% by mass or more, still further preferably 5% by mass or more, in view of imparting higher shape sustainability and strength to the treated human hair fibers, and preferably 60% by mass or less, more preferably 50% by mass or less, further preferably 40% by mass or less, still further preferably 35% by mass or less, still further preferably 30% by mass or less, from the viewpoint mentioned above as well as in view of formulation suitability.

Specifically, the content of the component (A) in the human hair fiber treatment agent of the present invention is preferably 0.1 to 60% by mass, more preferably 1 to 50% by mass, further preferably 2.5 to 40% by mass, still further preferably 5 to 35% by mass, still further preferably 5 to 30% by mass, in view of imparting higher shape sustainability and strength to the treated human hair fibers and in view of formulation suitability.

[Component (B): specific melamine derivative]

The component (B) is a melamine derivative represented by the formula (1):

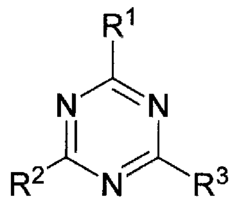

**(1)**

wherein $R^1$ to $R^3$ are the same or different and each represent a hydrogen atom, a hydroxymethylamino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms.

The melamine derivative as the component (B) is preferably at least one member selected from the group consisting of melamine and trimethylol melamine, more preferably melamine, in view of imparting higher shape sustainability and durability to the treated human hair fibers. These compounds as the component (B) can be used alone or in combination of two or more types thereof.

The content of the component (B) in the human hair fiber treatment agent of the present invention is preferably 0.1% by mass or more, more preferably 1% by mass or more, further preferably 2.5% by mass or more, still further preferably 5% by mass or more, in view of imparting higher shape sustainability and strength to the treated human hair fibers, and preferably 60% by mass or less, more preferably 50% by mass or less, further preferably 40% by mass or less, further preferably 35% by mass or less, still further preferably 30% by mass or less, from the viewpoint described above as well as in view of improving hair surface feel.

Specifically, the content of the component (B) in the human hair fiber treatment agent of the present invention is preferably 0.1 to 60% by mass, more preferably 1 to 50% by mass, further preferably 2.5 to 40% by mass, still further preferably 5 to 35% by mass, still further preferably 5 to 30% by mass, in view of imparting higher shape sustainability and strength to the treated human hair fibers and in view of improving hair surface feel.

The molar ratio of the component (B) to the component (A), which are applied to the human hair fibers by the human hair fiber treatment agent of the present invention, (B)/(A), is preferably 0.005 or more, more preferably 0.01 or more, further preferably 0.05 or more, still further preferably 0.1 or more, in view of attaining much better shape sustainability and strength of the treated human hair fibers through a condensate of the component (A) and the component (B) formed in the human hair fibers, and preferably less than 5, more preferably 4 or less, further preferably 3 or less, still further preferably 2 or less, in view of favorable feel.

Specifically, the molar ratio of the component (B) to the component (A), (B)/(A), is preferably 0.005 or more and less than 5, more preferably 0.01 to 4, further preferably 0.05 to 3, still further preferably 0.1 to 2, in view of attaining much better shape sustainability and durability of the treated human hair fibers through a condensate of the component (A) and the component (B) formed in the human hair fibers and in view of favorable feel.

[Component (C): water]

The human hair fiber treatment agent of the present invention comprises water as a vehicle. The content of the component (C) in the human hair fiber treatment agent of the present invention is preferably 10% by mass or more, more preferably 20% by mass or more, further preferably 30% by mass or more, still further preferably 40% by mass or more, and preferably 99% by mass or less, more preferably 97% by mass or less, further preferably 95% by mass or less, still further preferably 90% by mass or less.

Specifically, the content of the component (C) in the human hair fiber treatment agent of the present invention is preferably 10 to 99% by mass, more preferably 20 to 97% by mass, further preferably 30 to 95% by mass, still further preferably 40 to 90% by mass.

If necessary, a lower alcohol having 1 to 3 carbon atoms, such as methanol or ethanol, may be used in combination therewith. In this case, the content of the lower alcohol having 1 to 3 carbon atoms in the human hair fiber treatment agent of the present invention is preferably 60% by mass or less, more preferably 40% by mass or less, further preferably 30% by mass or less, still further preferably 20% by mass or less, further preferably 15% by mass or less, still further preferably 10% by mass or less, in view of imparting higher shape sustainability and durability to the treated human hair fibers. Also, the content is preferably 0.1% by mass or more.

The human hair fiber treatment agent of the present invention preferably further comprises glutaraldehyde in view of further improving the shape sustainability of the human hair fibers. The content of the glutaraldehyde in the human hair fiber treatment agent of the present invention is preferably 0.001% by mass or more, more preferably 0.05% by mass or more, further preferably 0.1% by mass or more, still further preferably 0.5% by mass or more, still further preferably 1.0% by mass or more, and preferably 30% by mass or less, more preferably 20% by mass or less, further preferably 15% by mass or less, still further preferably 10% by mass or less, still further preferably 5% by mass or less.

Specifically, the content of the glutaraldehyde in the human hair fiber treatment agent of the present invention is preferably 0.001 to 30% by mass, more preferably 0.05 to 20% by mass, further preferably 0.1 to 15% by mass, still further preferably 0.5 to 10% by mass, still further preferably 1.0 to 5% by mass.

The human hair fiber treatment agent of the present invention preferably contains a cationic surfactant in view of improving the feel of the treated human hair fibers and further improving the advantageous effects of the invention of the present application. The cationic surfactant is preferably a mono long-chain alkyl quaternary ammonium salt having one alkyl group having 8 to 24 carbon atoms and three alkyl groups having 1 to 4 carbon atoms.

Preferably, at least one mono long-chain alkyl quaternary ammonium surfactant is selected from the group

consisting of compounds represented by the following formula:

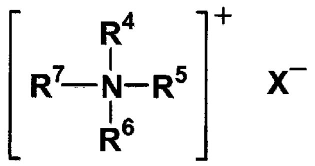

wherein $R^4$ represents a saturated or unsaturated linear or branched alkyl group having 8 to 22 carbon atoms, $R^8$-CO-NH-$(CH_2)_m$- or $R^8$-CO-O-$(CH_2)_m$- (wherein $R^8$ represents a saturated or unsaturated linear or branched alkyl chain having 7 to 21 carbon atoms, and m represents an integer of 1 to 4); $R^5$, $R^6$ and $R^7$ each independently represent an alkyl group having 1 to 4 carbon atoms or a hydroxylalkyl group having 1 to 4 carbon atoms; and $X^-$ represents a chloride ion, a bromide ion, a methosulfate ion or an ethosulfate ion.

Preferred examples of the cationic surfactant include long-chain quaternary ammonium compounds such as cetyl trimethyl ammonium chloride, myristyl trimethyl ammonium chloride, behentrimonium chloride, cetyl trimethyl ammonium bromide, and stearamidopropyltrimonium chloride. These cationic surfactants may be used alone or may be used as a mixture.

The content of the cationic surfactant in the human hair fiber treatment agent of the present invention is preferably 0.05% by mass or more, more preferably 0.1% by mass or more, and preferably 10% by mass or less, more preferably 5% by mass or less, in view of improving the feel of the treated human hair fibers and further improving the advantageous effects of the invention of the present application.

The human hair fiber treatment agent of the present invention preferably contains silicone in view of improving the feel of the treated human hair fibers and improving styling ease. The silicone is preferably one or more members selected from the group consisting of dimethylpolysiloxane and amino-modified silicone.

Any of cyclic or noncyclic dimethylpolysiloxane polymers can be used as the dimethylpolysiloxane. Examples thereof include SH200 series, BY22-019, BY22-020, BY11-026, B22-029, BY22-034, BY22-050A, BY22-055, BY22-060, BY22-083, and FZ-4188 (all from Dow Corning Toray Co., Ltd.), and KF-9088, KM-900 series, MK-15H, and MK-88 (all from Shin-Etsu Chemical Co., Ltd.).

Every silicone having an amino group or an ammonium group can be used as the amino-modified silicone. Examples thereof include amino-modified silicone oil with all or some terminal hydroxyl groups end-capped with a methyl group or the like, and amodimethicone without end capping. Preferred examples of the amino-modified silicone include compounds represented by the following formula, in view of improving the feel of the treated human hair fibers and improving styling ease:

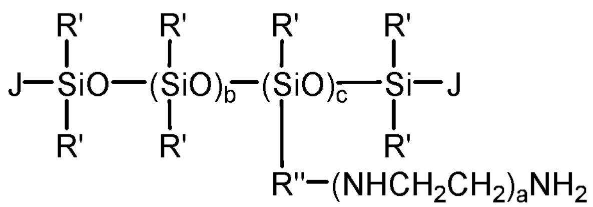

wherein R' represents a hydrogen atom, a hydroxy group or $R^x$; $R^x$ represents a substituted or unsubstituted monovalent hydrocarbon group having 1 to 20 carbon atoms; J represents $R^x$, R"-$(NHCH_2CH_2)_aNH_2$, $OR^x$ or a hydroxy group; R" represents a divalent hydrocarbon group having 1 to 8 carbon atoms; a represents a number of 0 to 3; and b and c represent numbers, the sum of which becomes 10 or more and less than 20,000, preferably 20 or more and less than 3,000, more preferably 30 or more and less than 1,000, further preferably 40 or more and less than 800, in terms of number average.

Specific examples of a preferred commercially available product of the amino-modified silicone include: amino-modified silicone oils such as SF8452C and SS3551 (both from Dow Corning Toray Co., Ltd.), and KF-8004, KF-867S, and KF-8015 (all from Shin-Etsu Chemical Co., Ltd.); and amodimethicone emulsions such as SM8704C, SM8904, BY22-079, FZ-4671, and FZ4672 (all from Dow Corning Toray Co., Ltd.).

The content of the silicone in the human hair fiber treatment agent of the present invention is preferably 0.1% by mass or more, more preferably 0.2% by mass or more, further preferably 0.5% by mass or more, and preferably 20% by mass or less, more preferably 10% by mass or less, further preferably 5% by mass or less, in view of improving the feel of the treated human hair fibers and further improving the advantageous effects of the invention of the present application.

The human hair fiber treatment agent of the present invention preferably contains a cationic polymer in view of improving the feel of the treated human hair fibers.

The cationic polymer refers to a polymer having a cationic group or a group ionizable with a cationic group and also includes an ampholytic polymer which becomes cationic as a whole. Specific examples thereof include aqueous solutions containing an amino group or an ammonium group at a side chain of the polymer chain, or containing diallyl quaternary ammonium salt as a constituent unit, for example, cationized cellulose derivatives, cationic starch, cationized guar gum derivatives, polymers or copolymers of diallyl quaternary ammonium salt, and quaternized polyvinylpyrrolidone derivatives. Among them, one or two or more members selected from the group consisting of a polymer containing diallyl quaternary ammonium salt as a constituent unit, a quaternized polyvinylpyrrolidone derivative, and a cationized cellulose derivative are preferred, and one or two or more members selected from the group consisting of a polymer or a copolymer of diallyl quaternary ammonium salt and a cationized cellulose derivative are more preferred, in view of improving effects of soft feel upon rinsing or upon shampooing, smoothness and finger combability, styling ease upon drying and moisture-retaining properties, and the stability of the part(s).

Preferred specific examples of the polymer or copolymer of diallyl quaternary ammonium salt include dimethyl diallyl ammonium chloride polymers (polyquaternium-6, for example, MERQUAT 100; Lubrizol Advanced Materials, Inc.), dimethyl diallyl ammonium chloride/acrylic acid copolymers (polyquaternium-22, for example, MERQUAT 280 and MERQUAT 295; Lubrizol Advanced Materials, Inc.), and dimethyl diallyl ammonium chloride/acrylamide copolymers (polyquaternium-7, for example, MERQUAT 550; Lubrizol Advanced Materials, Inc.).

Preferred specific examples of the quaternized polyvinylpyrrolidone derivative include polymers obtained by copolymerizing a vinylpyrrolidone copolymer with dimethylaminoethyl methacrylate (polyquaternium 11, for example, GAFQUAT 734, GAFQUAT 755, and GAFQUAT 755N (all from Ashland Inc.)).

Preferred specific examples of the cationized cellulose include polymers obtained by adding glycidyl trimethyl ammonium chloride to hydroxycellulose (polyquaternium 10, for example, REOGUARD G and REOGUARD GP (all from Lion Corporation.), and Polymer JR-125, Polymer JR-400, Polymer JR-30M, Polymer LR-400, and Polymer LR-30M (all from Amerchol Corp.)), and hydroxyethylcellulose dimethyl diallyl ammonium chloride (polyquaternium-4, for example, CELCOAT H-100 and CELCOAT L-200 (all from Akzo Nobel N.V.)).

The content of the cationic polymer in the human hair fiber treatment agent of the present invention is preferably 0.001% by mass or more, more preferably 0.01% by mass or more, further preferably 0.05% by mass or more, and preferably 20% by mass or less, more preferably 10% by mass or less, in view of improving the feel of the treated human hair fibers.

The human hair fiber treatment agent of the present invention can further contain an antioxidant such as ascorbic acid; and/or a pH adjuster such as sodium hydroxide, potassium hydroxide, phosphoric acid, or hydrochloric acid.

pH of the human hair fiber treatment agent of the present invention is preferably 6.0 or higher, more preferably 6.5 or higher, further preferably 7.0 or higher, in view of improving penetration into hair, and preferably 12.0 or lower, more preferably 11.5 or lower, further preferably 11.0 or lower, in view of suppressing hair damage. The pH according to the present invention is a value determined at 25°C.

Specifically, pH of the human hair fiber treatment agent of the present invention is preferably 6.0 to 12.0, more preferably 6.5 to 11.5, further preferably 7.0 to 11.0, in view of improving penetration into hair and in view of suppressing hair damage.

[Method for treating human hair fibers]

Human hair fibers can be treated by a method comprising the following step (i) using the human hair fiber treatment agent of the present invention, thereby imparting shape sustainability and high durability to the human hair fibers:

( i) the step of immersing human hair fibers having free ends at both ends in the human hair fiber treatment agent of the present invention under heating.

In the step (i), the human hair fibers to be immersed in the human hair fiber treatment agent may be dry or wet. The amount of the human hair fiber treatment agent to immerse the human hair fibers is, in terms of a bath ratio to the mass of the human hair fibers (the mass of the human hair fiber treatment agent / the mass of the human hair fibers), preferably 2 or more, more preferably 3 or more, further preferably 5 or more, further preferably 10 or more, further preferably 20 or more, and preferably 500 or less, more preferably 250 or less, further preferably 100 or less.

Specifically, the bath ratio is preferably 2 to 500, more preferably 3 to 250, further preferably 5 to 100, still further preferably 10 to 100, still further preferably 20 to 100.

In the step (i), the human hair fibers having free ends at both ends may be fixed with a curler or the like in advance and subsequently immersed in the human hair fiber treatment agent of the present invention under heating.

This can impart shape sustainability and high durability as well as the desired shape to the human hair fibers.

The immersing of the human hair fibers in the human hair fiber treatment agent in the step (i) is performed under heating, and this heating is performed by warming the human hair fiber treatment agent. This heating may be performed by immersing the human hair fibers in the human hair fiber treatment agent while heating the human hair fiber treatment agent, or may be performed by immersing the human hair fibers in the human hair fiber treatment agent having a low temperature, followed by heating. The temperature of the human hair fiber treatment agent is preferably 50°C or higher, more preferably 60°C or higher, further preferably 80°C or higher, for increasing the interaction of the component (A) and the component (B) with protein in the human hair fibers, and accelerating condensation reaction between the component (A) and the component (B) in the human hair fibers, thereby obtaining the advantageous effects of the present invention, and preferably lower than 100°C, more preferably 99°C or lower, for preventing operability in subsequent steps from being reduced due to human hair fibers tangled by the vigorous boiling of the treatment agent during heating.

The immersing time in the step (i) is appropriately selected depending on the heating temperature used, and is preferably 15 minutes or longer, more preferably 30 minutes or longer, further preferably 1 hour or longer, in view of allowing the human hair fiber treatment agent to penetrate and/or diffuse into the human hair fibers, and causing sufficient polymerization, and preferably 48 hours or shorter, more preferably 24 hours or shorter, further preferably 12 hours or shorter, for suppressing human hair fiber damage.

The step (i) is preferably performed in an environment where the evaporation of moisture is suppressed. Specific examples of the approach of suppressing the evaporation of moisture include a method of covering a container for the human hair fiber treatment agent with the human hair fibers immersed therein with a film-like substance, a cap, a lid, or the like made of a material impermeable to water vapor.

After the step (i), the human hair fibers may or may not be rinsed, and are preferably rinsed in view of preventing the feel of the human hair fibers from being reduced due to redundant polymerization products.

These treatments allow the components (A) and (B) to penetrate into the human hair fibers, presumably causing their interaction with protein in the human hair fibers. Also, a condensate of the component (A) and the component (B) is formed in the human hair fibers. Hence, the human hair fibers treated by the method of the present invention does not lose shape even when cleansed.

After the step (i), the following step (ii) may be further performed and can thereby further improve the shape sustainability of the human hair fibers:

(ii) the step of immersing the human hair fibers in a post-crosslinking agent comprising the following components (D) and (C) :

> (D): at least one formaldehyde derivative selected from the group consisting of formaldehyde, formaldehyde hydrate, glyoxylic acid, glyoxylic acid hydrate, glyoxylate, glyoxal, glyoxal hydrate, glutaraldehyde, and glutaraldehyde hydrate; and
> (C): water.

The content of the component (D) in the post-crosslinking agent is preferably 0.01% by mass or more, more preferably 0.1% by mass or more, further preferably 1% by mass or more, and preferably 60% by mass or less, more preferably 40% by mass, further preferably 20% by mass or less.

Specifically, the content of the component (D) in the post-crosslinking agent is preferably 0.01 to 60% by mass, more preferably 0.1 to 40% by mass, further preferably 1 to 20% by mass.

The post-crosslinking agent can further contain a pH adjuster such as sodium hydroxide, potassium hydroxide, phosphoric acid, hydrochloric acid, or an organic acid. On the other hand, the post-crosslinking agent is preferably free from the melamine derivative as the component (B) or a resorcinol derivative as a component (E) mentioned later in view of improving the surface feel of hair.

pH of the post-crosslinking agent is preferably 6.0 or lower, more preferably 5.0 or lower, further preferably 4.5 or lower, in view of improving penetration into hair, and preferably 0 or higher, more preferably 0.5 or higher, further preferably 1 or higher, in view of suppressing hair damage.

Specifically, pH of the post-crosslinking agent is preferably 0 to 6.0, more preferably 0.5 to 5.0, further preferably 1 to 4.5, in view of improving penetration into hair and in view of suppressing hair damage.

The temperature of the post-crosslinking agent for use in the step (ii) is preferably 40°C or higher, more preferably 60°C or higher, further preferably 80°C or higher, in view of increasing the interaction between a condensate of the component (A) and the component (B) formed in the human hair fibers and protein in the human hair fibers, and enhancing the advantageous effects of the present invention (shape sustainability and strength), and preferably lower than 100°C, more preferably 99°C or lower, in view of preventing operability in subsequent steps from being reduced due to human hair fibers tangled by the vigorous boiling of the treatment agent during heating.

In the step (ii), the human hair fibers to be immersed in the post-crosslinking agent may be dry or wet. The

amount of the post-crosslinking agent to immerse the human hair fibers is, in terms of a bath ratio to the mass of the human hair fibers (the mass of the post-crosslinking agent / the mass of the human hair fibers treated in the step (i)), preferably 2 or more, more preferably 3 or more, further preferably 5 or more, still further preferably 10 or more, still further preferably 20 or more, and preferably 500 or less, more preferably 250 or less, further preferably 100 or less.

Specifically, the bath ratio is preferably 2 to 500, more preferably 3 to 250, further preferably 5 to 100, still further preferably 10 to 100, still further preferably 20 to 100.

In the step (ii), the immersing time of the human hair fibers in the post-crosslinking agent is preferably 1 minute or longer, more preferably 3 minutes or longer, further preferably 5 minutes or longer, and preferably 5 hours or shorter, more preferably 3 hours or shorter, further preferably 1 hour or shorter, for allowing the post-crosslinking agent to penetrate and/or diffuse into the human hair fibers.

After the step (i) or (ii), the following step (iii) may be further performed and can thereby markedly improve hair surface feel:

(iii) the step of immersing the human hair fibers in a surface finish agent (I) comprising the following components (E) and (C):

(E): a resorcinol derivative represented by the formula (2):

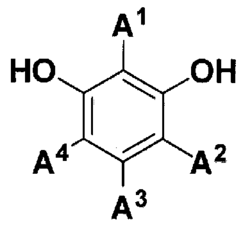

(2)

wherein

$A^1$ to $A^4$ are the same or different and each represent a hydrogen atom, a hydroxy group, a halogen atom, a carboxyl group or a salt thereof, a sulfonic acid group or a salt thereof, a linear or branched alkyl group or alkenyl group having 1 to 6 carbon atoms, or a linear or branched alkoxy group or alkenyloxy group having 1 to 6 carbon atoms; and

(C): water.

In this case, preferred examples of the component (E) include resorcinol, 2-methylresorcinol, 4-chlororesorcinol, and pyrogallol. The content of the component (E) in the surface finish agent (I) is preferably 1% by mass or more, more preferably 2.5% by mass or more, further preferably 5% by mass or more, still further preferably 10% by mass or more, still further preferably 20% by mass or more, and preferably 98% by mass or less, more preferably 97% by mass or less, further preferably 95% by mass or less, still further preferably 93% by mass or less, still further preferably 80% by mass or less, in view of improving hair surface feel.

The surface finish agent (I) can further contain a pH adjuster such as sodium hydroxide, potassium hydroxide, phosphoric acid, hydrochloric acid, or an organic acid. On the other hand, the surface finish agent (I) is preferably free from the melamine derivative as the component (B) or the formaldehyde derivative as the component (D) in view of improving the surface feel of hair.

pH of the surface finish agent (I) is preferably 7.0 or lower, more preferably 6.8 or lower, further preferably 6.5 or lower, in view of improving penetration into hair, and preferably 0 or higher, more preferably 0.5 or higher, further preferably 1.0 or higher, in view of suppressing hair damage.

Specifically, pH of the surface finish agent (I) is preferably 0 to 7.0, more preferably 0.5 to 6.8, further preferably 1.0 to 6.5, in view of improving penetration into hair and in view of suppressing hair damage.

The temperature of the surface finish agent (I) for use in the step (iii) is preferably 0°C or higher, more preferably 20°C or higher, further preferably 40°C or higher, and preferably 80°C or lower, more preferably 60°C or lower, in view of allowing the surface finish agent (I) to efficiently penetrate and/or diffuse to the inside of the human hair fibers, and further enhancing the effect of improving feel.

In the step (iii), the human hair fibers to be immersed in the surface finish agent (I) may be dry or wet. The amount of the surface finish agent to immerse the human hair fibers is, in terms of a bath ratio to the mass of the human hair fibers (the mass of the surface finish agent (I) / the mass of the human hair fibers treated in the step (ii)), preferably 2 or more, more preferably 3 or more, further preferably 5 or more, still further preferably 10 or more, still further preferably 20 or more, and preferably 500 or less, more preferably 250 or less, further preferably 100 or less.

Specifically, the bath ratio is preferably 2 to 500, more preferably 3 to 250, further preferably 5 to 100, still further preferably 10 to 100, still further preferably 20 to 100.

In the step (iii), the immersing time of the human hair fibers in the surface finish agent (I) is preferably 1 hour or longer, more preferably 3 hours or longer, further preferably 6 hours or longer, still further preferably 24 hours or longer, and preferably 1 month or shorter, more preferably 2 weeks or shorter, further preferably 10 days or shorter, still further preferably 168 hours or shorter, for allowing the surface finish agent (I) to penetrate and/or diffuse into the human hair fibers.

[Optional treatment to be further added]

The method for treating human hair fibers according to the present invention may additionally comprise one or more treatments selected from the group consisting of cuticle removal, bleaching, hair dyeing, and surface finish for hydrophobization and/or reduction in friction, in addition to the steps (i) to (iii) mentioned above.

In this respect, each treatment of cuticle removal, bleaching, and hair dyeing may be performed before or after the steps (i) to (iii) mentioned above or may be performed between the steps among the steps (i) to (iii). Also, a plurality of treatments may be added in combination. Any of the treatments may be performed first, except that the bleaching needs to be performed before the hair dyeing in the case of adding both bleaching and hair dyeing. A different treatment such as cuticle removal may be performed between the bleaching and the hair dyeing.

On the other hand, the surface finish for hydrophobization and/or reduction in friction needs to be performed after the step (i) mentioned above, or to be performed after the step (ii) when performed in combination with treatment by crosslinking agent, or to be performed after the step (iii) when performed in combination with surface finish for improvement in feel in the step (iii). Among others, the surface finish for hydrophobization and/or reduction in friction additionally performed after the step (iii) can produce more favorable results. The surface finish for hydrophobization and/or reduction in friction is not particularly limited by the order of treatments also including cuticle removal, bleaching, and hair dyeing as long as the surface finish is performed at a stage after the step (i), (ii) or (iii) as described above.

(Cuticle removal)

Cuticle present on human hair fiber surface has a directional structure where scales are piled. If human hair fibers having different directions coexist in one fiber tress, these human hair fibers get stuck with each other and are easily tangled, markedly impairing workability. Hence, the cuticle removal is performed in order to remove hair direction for easy handling, and performed by immersing the human hair fibers in a composition for cuticle removal given below.

The composition for cuticle removal contains a chlorine precursor having a cuticle peeling effect, and water. Examples of the chlorine precursor include sodium hypochlorite, potassium dichloroisocyanurate, sodium dichloroisocyanurate, and trichloroisocyanuric acid. One or more chlorine precursors selected from the group consisting of these compounds can be used.

The content of the chlorine precursor in the composition for cuticle removal is preferably 1% by mass or more, more preferably 2% by mass or more, and preferably 15% by mass or less, more preferably 12% by mass or less, further preferably 9% by mass or less.

pH at 25°C of the composition for cuticle removal is preferably 2 or higher, more preferably 3 or higher, further preferably 4.5 or higher, and preferably 10 or lower, more preferably 8 or lower, further preferably 7 or lower.

(Bleaching)

The bleaching is performed by immersing the human hair fibers in a bleach composition containing an alkali agent, an oxidizing agent and water. The bleach composition is usually of two-part type. The first part contains the alkali agent and water, and the second part contains the oxidizing agent and water. These two parts are usually stored separately and mixed before immersing of hair.

Preferred examples of the alkali agent include, but are not limited to: ammonia and salts thereof; alkanolamines (monoethanolamine, isopropanolamine, 2-amino-2-methylpropanol, 2-aminobutanol, etc.) and salts thereof; alkanediamines (1,3-propanediamine, etc.) and salts thereof; and carbonates (guanidine carbonate, sodium carbonate, potassium carbonate, sodium bicarbonate, potassium bicarbonate, etc.); and mixtures thereof.

The content of the alkali agent in the bleach composition (in the case of two-part type, in a mixture of the first part and the second part) is preferably 0.1% by mass or more, more preferably 0.5% by mass or more, further preferably 1% by mass or more, and preferably 15% by mass or less, more preferably 10% by mass or less, further preferably 7.5% by mass or less.

Preferred examples of the oxidizing agent include, but are not limited to, hydrogen peroxide, urea peroxide, melamine peroxide and sodium bromate. Among these oxidizing agents, hydrogen peroxide is preferred.

The content of the oxidizing agent in the bleach composition is preferably 1% by mass or more, more preferably 2% by mass or more, and preferably 15% by mass or less, more preferably 12% by mass or less, further preferably 9%

by mass or less.

In the case of separately storing the first part and the second part, pH at 25°C of the second part is preferably 2 or higher, more preferably 2.5 or higher, and preferably 6 or lower, more preferably 4 or lower. This pH can be adjusted with a suitable buffer. pH at 25°C of the bleach composition is preferably 6 or higher, more preferably 6.5 or higher, further preferably 6.8 or higher, and preferably 11 or lower, more preferably 10.5 or lower, further preferably 10 or lower.

(Hair dyeing)

The hair dyeing is performed by immersing the human hair fibers in a hair dye composition. The hair dye composition contains a dye and can optionally contain an alkali agent or an acid, an oxidizing agent, or the like. Examples of the dye include direct dyes, oxidizing dyes and combinations thereof.

The type of the direct dye is not particularly limited, and an arbitrary direct dye suitable for hair dyeing can be used. Examples of the direct dye include anionic dyes, nitro dyes, disperse dyes, cationic dyes, and dyes having an azophenol structure, selected from the group consisting of the following HC Red 18, HC Blue 18 and HC Yellow 16, and salts thereof, and mixtures thereof.

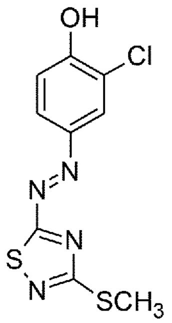

HC Red 18

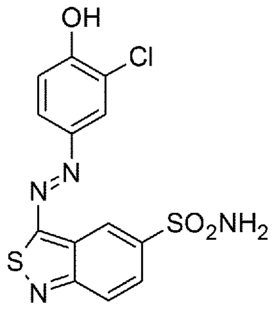

HC Blue 18

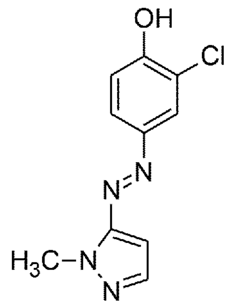

HC Yellow 16

Examples of the cationic dye include, but are not limited to, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12, Basic Red 22, Basic Red 51, Basic Red 76, Basic Violet 1, Basic Violet 2, Basic Violet 3, Basic Violet 10, Basic Violet 14, Basic Yellow 57 and Basic Yellow 87 and mixtures thereof. Basic Red 51, Basic Orange 31, Basic Yellow 87 and mixtures thereof are particularly preferred.

Examples of the anionic dye include, but are not limited to, Acid Black 1, Acid Blue 1, Acid Blue 3, Food Blue 5, Acid Blue 7, Acid Blue 9, Acid Blue 74, Acid Orange 3, Acid Orange 4, Acid Orange 6, Acid Orange 7, Acid Orange 10, Acid Red 1, Acid Red 14, Acid Red 18, Acid Red 27, Acid Red 33, Acid Red 50, Acid Red 52, Acid Red 73, Acid Red 87, Acid Red 88, Acid Red 92, Acid Red 155, Acid Red 180, Acid Violet 2, Acid Violet 9, Acid Violet 43, Acid Violet 49, Acid Yellow 1, Acid Yellow 10, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, and alkali metal salts (sodium salt, potassium salt, etc.) thereof and mixtures thereof.

Among them, the anionic dye is preferably selected from the group consisting of Acid Black 1, Acid Red 52, Acid Violet 2, Acid Violet 43, Acid Red 33, Acid Orange 4, Acid Orange 7, Acid Red 27, Acid Yellow 3 and Acid Yellow 10, and salts thereof. The anionic dye is more preferably selected from the group consisting of Acid Red 52, Acid Violet 2, Acid Red 33, Acid Orange 4 and Acid Yellow 10, and salts thereof and mixtures thereof.

Examples of the nitro dye include, but are not limited to, HC Blue No. 2, HC Blue No. 4, HC Blue No. 5, HC Blue No. 6, HC Blue No. 7, HC Blue No. 8, HC Blue No. 9, HC Blue No. 10, HC Blue No. 11, HC Blue No. 12, HC Blue No. 13, HC Brown No. 1, HC Brown No. 2, HC Green No. 1, HC Orange No. 1, HC Orange No. 2, HC Orange No. 3, HC Orange No. 5, HC Red BN, HC Red No. 1, HC Red No. 3, HC Red No. 7, HC Red No. 8, HC Red No. 9, HC Red No. 10, HC Red No. 11, HC Red No. 13, HC Red No. 54, HC Red No. 14, HC Violet BS, HC Violet No. 1, HC Violet No. 2, HC Yellow No. 2, HC Yellow No. 4, HC Yellow No. 5, HC Yellow No. 6, HC Yellow No. 7, HC Yellow No. 8, HC Yellow No. 9, HC Yellow No. 10, HC Yellow No. 11, HC Yellow No. 12, HC Yellow No. 13, HC Yellow No. 14, HC Yellow No. 15, 2-amino-6-chloro-4-nitrophenol, picramic acid, 1,2-diamino-4-nitrobenzole, 1,4-diamino-2-nitrobenzole, 3-nitro-4-aminophenol, 1-hydroxy-2-amino-3-nitrobenzole and 2-hydroxyethylpicramic acid and mixtures thereof.

Examples of the disperse dye include, but are not limited to, Disperse Blue 1, Disperse Black 9 and Disperse

Violet 1 and mixtures thereof.

These direct dyes can be used alone or in combination of two or more thereof, and direct dyes differing in ionicity may be used in combination.

The content of the direct dye in the hair dye composition is preferably 0.001% by mass or more, more preferably 0.01% by mass or more, further preferably 0.05% by mass or more, in view of obtaining sufficient hair dyeing properties, and preferably 10% by mass or less, more preferably 7.5% by mass or less, further preferably 5.0% by mass or less, further preferably 3.0% by mass or less, in view of blendability.

When the hair dye composition contains only a direct dye as the dye, no oxidizing agent is necessary for dyeing hair. In the case of lightening the color of hair, the composition may contain an oxidizing agent.

When the hair dye composition contains an oxidizing dye, the hair dye composition is usually of two-part type. The first part contains an oxidizing dye intermediate (precursor and coupler) and an alkali agent, and the second part contains an oxidizing agent such as hydrogen peroxide. These two parts are usually stored separately and mixed before immersing of hair.

The oxidizing dye intermediate is not particularly limited, and any known precursor and coupler usually used in hair dyeing products can be suitably used.

Examples of the precursor include, but are not limited to, paraphenylenediamine, toluene-2,5-diamine, 2-chloro-paraphenylenediamine, N-methoxyethyl-paraphenylenediamine, N-phenylparaphenylenediamine, N,N-bis(2-hydroxyethyl)-paraphenylenediamine, 2-(2-hydroxyethyl)-paraphenylenediamine, 2,6-dimethyl-paraphenylenediamine, 4,4'-diaminodiphenylamine, 1,3-bis(N-(2-hydroxyethyl)-N-(4-aminophenyl)amino)-2-propanol, PEG-3,3,2'-paraphenylenediamine, paraaminophenol, paramethylaminophenol, 3-methyl-4-aminophenol, 2-aminomethyl-4-aminophenol, 2-(2-hydroxyethylaminomethyl)-4-aminophenol, orthoaminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-hydroxypyrimidine, 4,5-diamino-1-(4'-chlorobenzyl)pyrazole, 4,5-diamino-1-hydroxyethylpyrazole, and salts of these substances and mixtures thereof.

Examples of the coupler include, but are not limited to, metaphenylenediamine, 2,4-diaminophenoxyethanol, 2-amino-4-(2-hydroxyethylamino)anisole, 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, metaaminophenol, 2-methyl-5-aminophenol, 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-metaaminophenol, 2-methyl-4-methoxy-5-(2-hydroxyethylamino)phenol, 2-methyl-4-fluoro-5-aminophenol, paraaminoorthocresol, resorcinol, 2-methylresorcinol, 4-chlororesorcinol, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, 2,6-diaminopyridine, and salts of these substances and mixtures thereof.

The content of each of the precursor and the coupler in the hair dye composition is preferably 0.01% by mass or more, more preferably 0.1% by mass or more, and preferably 10% by mass or less, more preferably 7.5% by mass or less, further preferably 5% by mass or less.

The hair dye composition containing an oxidizing dye further contains an alkali agent. Preferred examples of the alkali agent include, but are not limited to: ammonia and salts thereof; alkanolamines (monoethanolamine, isopropanolamine, 2-amino-2-methylpropanol, 2-aminobutanol, etc.) and salts thereof; alkanediamines (1,3-propanediamine, etc.) and salts thereof; and carbonates (guanidine carbonate, sodium carbonate, potassium carbonate, sodium bicarbonate, potassium bicarbonate, etc.); and mixtures thereof.

The content of the alkali agent in the hair dye composition is preferably 0.1% by mass or more, more preferably 0.5% by mass or more, further preferably 1% by mass or more, and preferably 15% by mass or less, more preferably 10% by mass or less, further preferably 7.5% by mass or less.

When the hair dye composition contains an oxidizing dye, the composition (second part) containing the oxidizing agent is stored separately from the composition (first part) containing the oxidizing dye, and mixed therewith before immersing of hair. Preferred examples of the oxidizing agent include, but are not limited to, hydrogen peroxide, urea peroxide, melamine peroxide and sodium bromate. Among these oxidizing agents, hydrogen peroxide is preferred.

The content of the oxidizing agent in the hair dye composition is preferably 1% by mass or more, more preferably 2% by mass or more, and preferably 15% by mass or less, more preferably 12% by mass or less, further preferably 9% by mass or less.

In the case of separately storing the first part and the second part, pH at 25°C of the second part is preferably 2 or higher, more preferably 2.5 or higher, and preferably 6 or lower, more preferably 4 or lower. This pH can be adjusted with a suitable buffer. pH at 25°C of the hair dye composition prepared by mixing the first part and the second part is preferably 6 or higher, more preferably 6.5 or higher, further preferably 6.8 or higher, and preferably 11 or lower, more

preferably 10.5 or lower, further preferably 10 or lower.

The hair dye composition containing an oxidizing dye may further contain the direct dye listed above.

The hair dye composition can preferably further contain a surfactant, a conditioning component, or the like given below, and can preferably be in any of solution, emulsion, cream, paste and mousse forms.

The temperature of the hair dye composition is preferably 0°C or higher, more preferably 10°C or higher, further preferably 20°C or higher, and preferably 90°C or lower, more preferably 80°C or lower, in view of allowing the hair dye composition to efficiently penetrate and/or diffuse to the inside of the human hair fibers, and further enhancing the effects of hair dyeing.

(Surface finish for hydrophobization and/or reduction in friction)

The surface finish for hydrophobization and/or reduction in friction is performed by immersing hair in a surface finish agent (II) given below at a stage after the step (i) mentioned above, or after the step (ii) when performed in combination with treatment by crosslinking agent, or after the step (iii) when performed in combination with surface finish for improvement in feel in the step (iii).

The surface finish agent (II) contains 0.01% by mass or more and 5.00% by mass or less of the following component (F), and water:

(F) an epoxyaminosilane copolymer which is a reaction product of the following compounds (a) to (d):

(a) polysiloxane having at least two oxiranyl groups or oxetanyl groups;
(b) polyether having at least two oxiranyl groups or oxetanyl groups;
(c) aminopropyltrialkoxysilane: and
(d) a compound selected from the group consisting of the following primary and secondary amines:

primary amine: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminopropyldiethylamine, benzylamine, naphthylamine, 3-amino-9-ethylcarbazole, 1-aminoheptafluorohexane, and 2,2,3,3,4,4,5,5,6,6,7,7,8,8,8-pentadecafluoro-1-octanamine; and
secondary amine: methylethylamine, methyloctadecylamine, diethanolamine, dibenzylamine, dihexylamine dicyclohexylamine, piperidine, pyrrolidine phthalimide, and polymer amine.

[Component (F): epoxyaminosilane copolymer]

The epoxyaminosilane copolymer as the component (F) is a reaction product of the following compounds (a) to (d).

<Compounds (a) and (b)>

The compound (a) is polysiloxane containing at least two oxiranyl groups or oxetanyl groups. Examples thereof include compounds represented by the following formula (5):

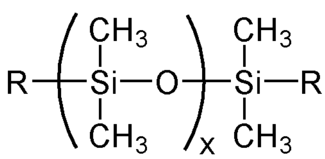

(5)

wherein R represents a hydrocarbon group having 1 to 6 carbon atoms and terminally having an oxiranyl group or an oxetanyl group, and optionally containing a heteroatom; and x represents a number of 1 to 1,000.

The compound (b) is polyether containing at least two oxiranyl groups or oxetanyl groups. Examples thereof include compounds represented by the following formula (6):

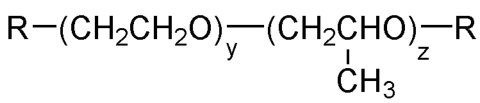

(6)

wherein R is as defined above; y represents a number of 1 to 100; z represents a number of 0 to 100; and y + z is 1 to 200.

In the formulas (5) and (6), the heteroatom optionally contained in R is preferably an oxygen atom. Examples of R include an oxiranylmethyl group (glycidyl group), an oxiranylmethoxy group (glycidyloxy group), an oxiranylmethoxypropyl group (glycidyloxypropyl group), an oxetanylmethyl group, an oxetanylmethoxy group, an oxetanylmethoxypropyl group, and a 3-ethyloxetanylmethyl group. Among them, a hydrocarbon group having 1 to 4 carbon atoms and having an oxiranyl group, and optionally containing a hetero oxygen atom is preferred, and at least one member selected from the group consisting of an oxiranylmethyl group (glycidyl group), an oxiranylmethoxy group (glycidyloxy group), and an oxiranylmethoxypropyl group (glycidyloxypropyl group) is further preferred.

<Compound (c)>

The compound (c) is aminopropyltrialkoxysilane. Examples of the alkoxy group in the compound (c) include alkoxy groups having 1 to 6 carbon atoms. An alkoxy group having 2 to 4 carbon atoms, further preferably an alkoxy group having 3 carbon atoms, is preferred. Among them, an isopropoxy group is preferred. Examples of the compound (c) include aminopropyltrimethoxysilane, aminopropyltriethoxysilane, aminopropyltripropoxysilane, aminopropyltriisopropoxysilane, aminopropyltributoxysilane, and aminopropyl tri-tert-butoxysilane. Among them, aminopropyltriisopropoxysilane is preferred. Any of these compounds (c) can be used alone or in combination of two or more thereof.

<Compound (d)>

The compound (d) is a compound selected from the group consisting of the following primary and secondary amines:

primary amine: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminoethyldimethylamine, aminoethyldiethylamine, aminoethyldibutylamine, aminopropyldimethylamine, aminopropyldiethylamine, aminopropyldibutylamine, benzylamine, naphthylamine, 3-amino-9-ethylcarbazole, 1-aminoheptafluorohexane, and 2,2,3,3,4,4,5,5,6,6,7,7,8,8,8-pentadecafluoro-1-octanamine; and
secondary amine: methylethylamine, methyloctadecylamine, diethanolamine, dibenzylamine, dihexylamine dicyclohexylamine, piperidine, pyrrolidine phthalimide, and polymer amine.

Among them, primary amine is preferred, and one member selected from the group consisting of aminopropyldiethylamine, aminopropyldimethylamine, and aminopropyldibutylamine is further preferred. Any of these compounds (d) can be used alone or in combination of two or more thereof.

The reaction of the compounds (a) to (d) is performed, for example, by refluxing these compounds for a given time in a solvent such as isopropanol. In this context, the molar ratio of the oxiranyl groups or the oxetanyl groups of the compounds (a) and (b) to the amino group of the compound (c) is preferably 1 or more, more preferably 1.1 or more, further preferably 1.2 or more, and preferably 4 or less, more preferably 3.9 or less, further preferably 3.8 or less.

Examples of the component (F) include compounds under INCI name of polysilicone-29. Examples of a commercially available product thereof include Silsoft CLX-E from Momentive Performance Materials, Inc. (active component: 15% by mass, containing dipropylene glycol and water).

The content of the component (F) in the surface finish agent (II) is 0.01% by mass or more, preferably 0.05% by mass or more, more preferably 0.10% by mass or more, further preferably 0.20% by mass or more, in view of imparting sufficient hydrophobicity to the human hair fibers, and 5.00% by mass or less, preferably 4.00% by mass or less, more preferably 3.00% by mass or less, further preferably 2.00% by mass or less, in view of offering no sticky feel.

[pH adjuster]

pH at 25°C of the surface finish agent (II) is preferably in a range given below in view of enhancing the reaction rate of the trialkoxysilane moiety of the component (F) in an acidic region or a basic region. When the surface finish agent (II) has a pH in an acidic region, pH is preferably 1 or higher, more preferably 1.5 or higher, further preferably 2 or higher, and preferably 5 or lower, more preferably 4.0 or lower, further preferably 3.5 or lower. When the surface finish agent (II) has a pH in a basic region, pH is preferably 7 or higher, more preferably 7.5 or higher, further preferably 8.0 or higher, and preferably 11 or lower, more preferably 10.5 or lower, further preferably 10 or lower. In order to adjust pH of the surface finish agent (II) to the range described above, the surface finish agent (II) can appropriately contain a pH adjuster. Examples of the pH adjuster that can be used include alkali agents including: alkanolamines such as monoethanolamine, isopropanolamine, 2-amino-2-methylpropanol, and 2-aminobutanol, or salts thereof; alkanediamines such as 1,3-propanediamine, or salts thereof; carbonates such as guanidine carbonate, sodium carbonate, potassium carbonate, sodium bicarbonate, and potassium bicarbonate; and hydroxides such as sodium hydroxide and potassium

hydroxide. Examples of the pH adjuster that can be used include acid agents including: inorganic acids such as hydrochloric acid and phosphoric acid; hydrochlorides such as monoethanolamine hydrochloride; phosphates such as monopotassium dihydrogen phosphate, and disodium monohydrate phosphate; and organic acids such as lactic acid and malic acid.

The amount of the surface finish agent (II) to immerse the human hair fibers is, in terms of a bath ratio to the mass of the human hair fibers (the mass of the surface finish agent (II) / the mass of the human hair fibers), preferably 2 or more, more preferably 5 or more, further preferably 10 or more, and preferably 100 or less, more preferably 50 or less, further preferably 20 or less.

The treatment of the human hair fibers by the method for treating human hair fibers described above can produce human hair fibers for wigs which are excellent in shape sustainability and durability while retaining the natural appearance of human hair. Also, a wig can be produced using the fibers.

The treatment of the human hair fibers by the method for treating human hair fibers according to the present invention can produce human hair fibers for hair extensions which are excellent in shape sustainability and durability while retaining the natural appearance of human hair. Also, hair extensions can be produced using the fibers.

The following aspects are preferred for the treatment agent capable of producing human hair fibers for wigs which are excellent in shape sustainability and durability while retaining the natural appearance and good feel of human hair, and a treatment method using the treatment agent.

[Treatment agent 1]

A human hair fiber treatment agent for treating human hair fibers having free ends at both ends, comprising the following components (A) to (C) in the formulation thereof and having pH of 7.0 to 11:

(A) 1.5 to 15% by mass of formaldehyde;
(B) 4.2 to 42% by mass of melamine; and
(C) a balance of water.

[Treatment agent kit 1]

A human hair fiber treatment agent kit for treating human hair fibers having free ends at both ends, comprising treatment agent 1 and a post-crosslinking agent having pH of 1.0 to 4.5, wherein the post-crosslinking agent comprises the following components (D) and (C):

(D) 1 to 20% by mass of glyoxylic acid or formaldehyde; and
(C) a balance of water.

[Treatment agent kit 1']

The human hair fiber treatment agent kit, wherein the treatment agent kit 1 further comprises a surface finish agent comprising the following components (E) and (C) and having pH of 1.0 to 6.5:

(E) 20 to 60% by mass of resorcinol; and
(C) a balance of water.

[Treatment method 1]

A method for treating human hair fibers, comprising the following step (i):

(i) the step of immersing human hair fibers having free ends at both ends in a human hair fiber treatment agent under heating of 90 to 99°C for 1 to 3 hours at a bath ratio of (human hair fiber treatment agent mass) / (human hair fiber mass) = 20 to 100, wherein the human hair fiber treatment agent comprises the following components (A) to (C) in the formulation thereof and has pH of 7.0 to 11.0:

(A) 1.5 to 15% by mass of formaldehyde;
(B) 4.2 to 42% by mass of melamine; and
(C) a balance of water.

[Treatment method 1']

The method for treating human hair fibers, wherein the treatment method 1 further comprises the following step (ii) after the step (i):
(ii) the step of immersing the human hair fibers treated in the step (i) in a post-crosslinking agent at a temperature of 90 to 90°C for 1 to 3 hours at a bath ratio of (post-crosslinking agent mass) / (the mass of the human hair fibers treated in the step (i)) = 20 to 100, wherein
the post-crosslinking agent comprises the following components (D) and (C) and has pH of 1.0 to 4.5:

(D) 1 to 20% by mass of glyoxylic acid or formaldehyde; and
(C) a balance of water.

[Treatment method 1']

The method for treating human hair fibers, wherein the treatment method 1 further comprises the following step (iii) after the step (ii):
(iii) the step of immersing the human hair fibers treated in the step (ii) in a surface finish agent (I) at a temperature of 20 to 90°C for 24 to 168 hours at a bath ratio of (the mass of the surface finish agent (I)) / (the mass of the human hair fibers treated in the step (ii)) = 20 to 100, wherein
the surface finish agent (I) comprises the following components (E) and (C) and has pH of 1.0 to 6.5:

(E) 20 to 60% by mass of resorcinol; and
(C) a balance of water.

Hereinafter, preferred aspects of the present invention will be further disclosed as to the embodiments mentioned above.

<1> A human hair fiber treatment agent for treating human hair fibers having free ends at both ends, comprising the following components (A) to (C) in the formulation thereof:

(A): formaldehyde or hydrate thereof;
(B): melamine derivative represented by the formula (1):

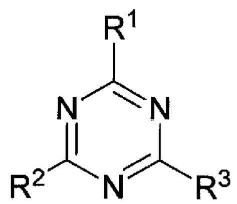

(1)

wherein $R^1$ to $R^3$ are the same or different and each represent a hydrogen atom, a hydroxymethylamino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms; and
(C): water.

<2> The human hair fiber treatment agent according to <1>, wherein the content of the component (A) is preferably 0.1% by mass or more, more preferably 1% by mass or more, further preferably 2.5% by mass or more, still further preferably 5% by mass or more, and preferably 60% by mass or less, more preferably 50% by mass or less, further preferably 40% by mass or less, still further preferably 35% by mass or less, still further preferably 30% by mass or less.
<3> The human hair fiber treatment agent according to <1> or <2>, wherein the component (B) is preferably at least one member selected from the group consisting of melamine and trimethylol melamine, more preferably melamine.
<4> The human hair fiber treatment agent according to any one of <1> to <3>, wherein the content of the component (B) is preferably 0.1% by mass or more, more preferably 1% by mass or more, further preferably 2.5% by mass or more, still further preferably 5% by mass or more, and preferably 60% by mass or less, more preferably 50% by mass or less, further preferably 40% by mass or less, still further preferably 35% by mass or less, still further preferably 30% by mass or less.

<5> The human hair fiber treatment agent according to any one of <1> to <4>, wherein the molar ratio of the component (B) to the component (A), (B)/(A) is preferably 0.005 or more, more preferably 0.01 or more, further preferably 0.05 or more, still further preferably 0.1 or more, and preferably less than 5, more preferably 4 or less, further preferably 3 or less, still further preferably 2 or less.
<6> The human hair fiber treatment agent according to any one of <1> to <5>, wherein the content of the component (C) is preferably 10% by mass or more, more preferably 20% by mass or more, further preferably 30% by mass or more, still further preferably 40% by mass or more, and preferably 99% by mass or less, more preferably 97% by mass or less, further preferably 95% by mass or less, still further preferably 90% by mass or less.
<7> The human hair fiber treatment agent according to any one of <1> to <6>, preferably further comprising glutaraldehyde.
<8> The human hair fiber treatment agent according to <7>, wherein the content of the glutaraldehyde is preferably 0.001% by mass or more, more preferably 0.05% by mass or more, further preferably 0.1% by mass or more, still further preferably 0.5% by mass or more, still further preferably 1.0% by mass or more, and preferably 30% by mass or less, more preferably 20% by mass or less, further preferably 15% by mass or less, still further preferably 10% by mass or less, still further preferably 5% by mass or less.
<9> The human hair fiber treatment agent according to any one of <1> to <8>, wherein pH at 25°C is preferably 6.0 or higher, more preferably 6.5 or higher, further preferably 7.0 or higher, and preferably 12.0 or lower, more preferably 11.5 or lower, further preferably 11.0 or lower.
<10> A method for treating human hair fibers, comprising the following step (i):

(i) the step of immersing human hair fibers having free ends at both ends in a human hair fiber treatment agent according to any of <1> to <9> under heating.

<11> The method for treating human hair fibers according to <10>, wherein the bath ratio (human hair fiber treatment agent mass) / (human hair fiber mass) in immersing the human hair fibers in the human hair fiber treatment agent is preferably 2 or more, more preferably 3 or more, further preferably 5 or more, still further preferably 10 or more, still further preferably 20 or more, and preferably 500 or less, more preferably 250 or less, further preferably 100 or less.
<12> The method for treating human hair fibers according to <10> or <11>, wherein the temperature of the human hair fiber treatment agent to immerse the human hair fibers is preferably 50°C or higher, more preferably 60°C or higher, further preferably 80°C or higher, and preferably lower than 100°C, more preferably 99°C or lower.
<13> The method for treating human hair fibers according to any one of <10> to <12>, wherein the immersing time of the human hair fibers in the human hair fiber treatment agent is preferably 15 minutes or longer, more preferably 30 minutes or longer, further preferably 1 hour or longer, and preferably 48 hours or shorter, more preferably 24 hours or shorter, further preferably 12 hours or shorter.
<14> The method for treating human hair fibers according to any one of <10> to <13>, preferably further comprising the following step (ii) after the step (i):
(ii) the step of immersing the human hair fibers in a post-crosslinking agent comprising the following components (D) and (C) :

(D): at least one formaldehyde derivative selected from the group consisting of formaldehyde, formaldehyde hydrate, glyoxylic acid, glyoxylic acid hydrate, glyoxylate, glyoxal, glyoxal hydrate, glutaraldehyde, and glutaraldehyde hydrate; and
(C): water.

<15> The method for treating human hair fibers according to <14>, wherein the content of the component (D) in the post-crosslinking agent is preferably 0.01% by mass or more, more preferably 0.1% by mass or more, further preferably 1% by mass or more, and preferably 60% by mass or less, more preferably 40% by mass, further preferably 20% by mass or less.
<16> The method for treating human hair fibers according to <14> or <15>, wherein pH at 25°C of the post-crosslinking agent is preferably 6.0 or lower, more preferably 5.0 or lower, further preferably 4.5 or lower, and preferably 0 or higher, more preferably 0.5 or higher, further preferably 1 or higher.
<17> The method for treating human hair fibers according to any one of <14> to <16>, wherein the temperature of the post-crosslinking agent is preferably 40°C or higher, more preferably 60°C or higher, further preferably 80°C or higher, and preferably lower than 100°C, more preferably 99°C or lower.
<18> The method for treating human hair fibers according to any one of <14> to <17>, wherein the bath ratio (the mass of the post-crosslinking agent / the mass of the human hair fibers treated in the step (i)) in immersing the human hair fibers in the post-crosslinking agent is preferably 2 or more, more preferably 3 or more, further preferably 5 or more, still further preferably 10 or more, still further preferably 20 or more, and preferably 500 or less, more

preferably 250 or less, further preferably 100 or less.

<19> The method for treating human hair fibers according to any one of <14> to <18>, wherein the immersing time of the human hair fibers in the post-crosslinking agent is preferably 1 minute or longer, more preferably 3 minutes or longer, further preferably 5 minutes or longer, and preferably 5 hours or shorter, more preferably 3 hours or shorter, further preferably 1 hour or shorter.

<20> The method for treating human hair fibers according to any one of <14> to <19>, preferably further comprising the following step (iii) after the step (i) or (ii):

(iii) the step of immersing the human hair fibers in a surface finish agent (I) comprising the following components (E) and (C):

(E): a resorcinol derivative represented by the formula (2):

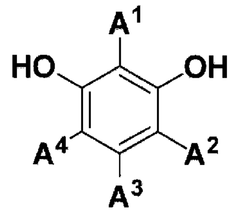

(2)

wherein

$A^1$ to $A^4$ are the same or different and each represent a hydrogen atom, a hydroxy group, a halogen atom, a carboxyl group or a salt thereof, a sulfonic acid group or a salt thereof, a linear or branched alkyl group or alkenyl group having 1 to 6 carbon atoms, or a linear or branched alkoxy group or alkenyloxy group having 1 to 6 carbon atoms; and

(C): water.

<21> The method for treating human hair fibers according to <20>, wherein the component (E) is one or more members selected from the group consisting of resorcinol, 2-methylresorcinol, 4-chlororesorcinol and pyrogallol.

<22> The method for treating human hair fibers according to <20> or <21>, wherein the content of the component (E) in the surface finish agent (I) is preferably 1% by mass or more, more preferably 2.5% by mass or more, further preferably 5% by mass or more, still further preferably 10% by mass or more, still further preferably 20% by mass or more, and preferably 98% by mass or less, more preferably 97% by mass or less, further preferably 95% by mass or less, still further preferably 93% by mass or less, still further preferably 80% by mass or less.

<23> The method for treating human hair fibers according to any one of <20> to <22>, wherein pH at 25°C of the surface finish agent (I) is preferably 7.0 or lower, more preferably 6.8 or lower, further preferably 6.5 or lower, and preferably 0 or higher, more preferably 0.5 or higher, further preferably 1.0 or higher.

<24> The method for treating human hair fibers according to any one of <20> to <23>, wherein the temperature of the surface finish agent (I) is preferably 0°C or higher, more preferably 20°C or higher, further preferably 40°C or higher, and preferably 80°C or lower, more preferably 60°C or lower.

<25> The method for treating human hair fibers according to any one of <20> to <24>, wherein the bath ratio (the mass of the surface finish agent / the mass of the human hair fibers treated in the step (ii)) in immersing the human hair fibers in the surface finish agent (I) is preferably 2 or more, more preferably 3 or more, further preferably 5 or more, still further preferably 10 or more, still further preferably 20 or more, and preferably 500 or less, more preferably 250 or less, further preferably 100 or less.

<26> The method for treating human hair fibers according to any one of <20> to <25>, wherein the immersing time of the human hair fibers in the surface finish agent (I) is preferably 1 hour or longer, more preferably 3 hours or longer, further preferably 6 hours or longer, still further preferably 24 hours or longer, and preferably 1 month or shorter, more preferably 2 weeks or shorter, further preferably 10 days or shorter, still further preferably 168 hours or shorter.

<27> A method for producing a wig, comprising the step of treating human hair fibers for wigs by a method for treating human hair fibers according to any one of <10> to <26>.

<28> A human hair fiber treatment agent for treating human hair fibers having free ends at both ends, comprising the following components (A) to (C) in the formulation thereof and having pH of 7.0 to 11:

(A) 1.5 to 15% by mass of formaldehyde;
(B) 4.2 to 42% by mass of melamine; and
(C) a balance of water.

<29> A human hair fiber treatment agent kit for treating human hair fibers having free ends at both ends, comprising a human hair fiber treatment agent according to <28> and a post-crosslinking agent comprising the following components (D) and (C) and having pH of 1.0 to 4.5:

(D) 1 to 20% by mass of glyoxylic acid or formaldehyde; and
(C) a balance of water.

<30> The human hair fiber treatment agent kit according to <29>, preferably further comprising a surface finish agent (I) comprising the following components (E) and (C) and having pH of 1.0 to 6.5:

(E) 20 to 60% by mass of resorcinol; and
(C) a balance of water.

<31> A method for treating human hair fibers, comprising the following step (i):

(i) the step of immersing human hair fibers having free ends at both ends in a human hair fiber treatment agent under heating of 90 to 99°C for 1 to 3 hours at a bath ratio of (human hair fiber treatment agent mass) / (human hair fiber mass) = 20 to 100, wherein
the human hair fiber treatment agent comprises the following components (A) to (C) in the formulation thereof and has pH of 7.0 to 11.0:

(A) 1.5 to 15% by mass of formaldehyde;
(B) 4.2 to 42% by mass of melamine; and
(C) a balance of water.

<32> The method for treating human hair fibers according to <31>, preferably further comprising the following step (ii) after the step (i):
(ii) the step of immersing the human hair fibers treated in the step (i) in a post-crosslinking agent at a temperature of 90 to 90°C for 1 to 3 hours at a bath ratio of (post-crosslinking agent mass) / (the mass of the human hair fibers treated in the step (i)) = 20 to 100, wherein
the post-crosslinking agent comprises the following components (D) and (C) and has pH of 1.0 to 4.5:

(D) 1 to 20% by mass of glyoxylic acid or formaldehyde; and
(C) a balance of water.

<33> The method for treating human hair fibers according to <32>, preferably further comprising the following step (iii) after the step (ii):
(iii) the step of immersing the human hair fibers treated in the step (ii) in a surface finish agent (I) at a temperature of 20 to 90°C for 24 to 168 hours at a bath ratio of (the mass of the surface finish agent (I)) / (the mass of the human hair fibers treated in the step (ii)) = 20 to 100, wherein
the surface finish agent (I) comprises the following components (E) and (C) and has pH of 1.0 to 6.5:

(E) 20 to 60% by mass of resorcinol; and
(C) a balance of water.

Examples

Examples 1 to 5, and Comparative Examples 1 to 3 (Treatment with human hair fiber treatment agent in one-part form)

Each treatment agent shown in Table 1 was prepared and used in treatments given below to evaluate its shape sustainability, good surface feel, and durability. The results are also shown in Table 1. pH of each composition was measured directly from the prepared composition at room temperature (25°C) using a pH meter (manufactured by HORIBA, Ltd., F-52).

<Treatment with human hair fiber treatment agent>

1. A 25 cm long tress consisting of 0.5 g of Caucasian hair (untreated hair / almost straight hair with very slight wave) was immersed in 40 g of the human hair fiber treatment agent in a container. The resulting container was hermetically sealed by covering the opening of the container with plastic wrap, and heated for 3 hours in an oven (Drying Oven Forced Convection System with Window Stainless; manufactured by AS ONE Corporation, SOFW-450) set to 90°C.
2. The container containing the tress was taken out of the oven and cooled to room temperature.
3. The tress was taken out of the container, rinsed under running tap water of 30°C for 30 seconds, washed with foams of shampoo for evaluation for 60 seconds, rinsed under running tap water of 30°C for 30 seconds, and slightly towel-dried. Then, the tress was dried with a warm-air dryer (manufactured by TESCOM & Co., Ltd., Nobby White NB3000). At this point in time, the tress maintained straight hair.

<Formulation of shampoo for evaluation>

| Component | (% by mass) |
|---|---|
| Sodium laureth sulfate | 15.5 |
| Lauramide DEA | 1.5 |
| Sodium benzoate | 0.5 |
| EDTA-2Na | 0.3 |
| Phosphoric acid amount necessary for pH adjustment to 7 | |
| Deionized water | balance |
| Total | 100.0 |

<Shape sustainability>

1. The tress (straight hair) subjected to <Treatment with human hair fiber treatment agent> was wetted with tap water of 30°C for 30 seconds. Then, the wet tress was wrapped around a plastic rod having a diameter of 14 mm and fixed with a clip.
2. The whole rod was hermetically wrapped by covering with plastic wrap, and heated for 1 hour in an oven set to 90°C.
3. The tress was taken out of the oven and cooled to room temperature.
4. The tress was removed from the rod, rinsed under running tap water of 30°C for 30 seconds, and washed with foams of shampoo for evaluation for 60 seconds.
5. The tress was rinsed under running tap water of 30°C for 30 seconds, immersed in tap water of 30°C for 60 seconds at an infinite bath ratio, then gently pulled out of water by holding the root of the tress, and drained by light shaking.
6. The tress was left standing by hanging for 2 hours in a laboratory, dried, and combed. Then, the tress was hung and photographed from the side. On the basis of the photograph, the curvature radius (r) and curvature (1/r) of a most strongly curled part of the tress were calculated.

(Evaluation criteria)

The curvature of untreated hair was defined as $1/r_0$, and the curvature measured by step 6 described above was defined as 1/r. The rate (I) of increase in curvature (%) determined according to an expression given below was defined as shape sustainability (as the curled shape conferred by wrapping around a rod and heating is sustained as strong curl after hair wash, the value of I gets larger; the larger value of I means that the shape once conferred lasts longer, i.e., the shape sustainability is higher).

$$I = [(1/r) / (1/r_0)] \times 100 \ [\%].$$

<Good surface feel>

For the evaluation of feel, the tress immediately after evaluation of <Shape sustainability> was used and evaluated for its smooth feel when touched by hands, by 5 dedicated panelists according to the criteria given below. A numeric value obtained by rounding off a mean from the 5 panelists was used as evaluation results.

(Evaluation criteria)

5: very smooth hand feeling as compared with untreated hair.
4: smooth hand feeling as compared with untreated hair.
3: slightly smooth hand feeling as compared with untreated hair.
2: very slightly smooth hand feeling as compared with untreated hair.
1: the same hand feeling as in untreated hair.

<Durability>

For the evaluation of durability, the tress immediately after evaluation of <Shape sustainability> was used, and the tensile modulus of elasticity (Young's modulus) was used as an index for the durability of human hair fibers. The evaluation was conducted by the following steps.

1. Five human hair fibers were cut out from the root of the tress. A 3 cm human hair fiber specimen was collected from around the intermediate between the root and tip of each human hair fiber to obtain a total of ten 3 cm hair specimens. Each human hair fiber specimen was left in a room of 20°C and 60% RH for humidity condition for 24 hours.

2. The human hair fiber specimen was set in "MTT690 Automated Fiber Tensile Tester" manufactured by DIA-STRON limited. Automated measurement was started to determine the tensile modulus of elasticity (Young's modulus) in a wet state of the human hair fiber. The tensile modulus of elasticity (Young's modulus) shown in the tables was determined from a mean of the measurement results at N = 5. A higher numeric value means better strength as well as better durability.

[Table 1]

| Component (% by mass) | | | Example 1 | Example 2 | Example 3 | Example 4 | Example 5 | Comparative Example 1(*) | Comparative Example 2 | Comparative Example 3 |
|---|---|---|---|---|---|---|---|---|---|---|
| Human hair fiber treatment agent | (A) | Formaldehyde | 5 | 1.5 | 15 | 10 | 5 | Not treated | 5 | - |
| | (B) | Melamine | 14 | 4.2 | 42 | 14 | 14 | | - | 14 |
| | (C) | Water | Balance | Balance | Balance | Balance | Balance | | Balance | Balance |
| | Others | Sodium hydroxide | (**) | (**) | (**) | (**) | (**) | | (**) | (**) |
| | Total | | 100 | 100 | 100 | 100 | 100 | | 100 | 100 |
| | pH (25°C) | | 9 | 9 | 9 | 9 | 9 | | 9 | 9 |
| | Molar ratio (A)/(B) | | 1.5 | 1.5 | 1.5 | 3 | 1.5 | | - | - |
| | Bath ratio | | 80 | 80 | 80 | 80 | 4 | | 80 | 80 |
| Evaluation | Shape sustainability (value of I) | | 250 | 152 | 263 | 227 | 167 | 100 | 102 | 100 |
| | Good hair surface feel | | 3 | 3 | 2 | 2 | 2 | 1 | 1 | 1 |
| | Durability (Young's modulus [GPa]) | | 2.14 | 1.94 | 2.39 | 2.30 | 2.04 | 1.86 | 1.83 | 1.74 |

*: untreated hair
**: amount for pH adjustment

Examples 6 to 8 (Treatment with a human hair fiber treatment agent and a post treatment agent)

Each treatment agent shown in Table 2 was prepared and used in treatments given below to evaluate its shape sustainability, good surface feel, and durability according to the same method and criteria as in Examples 1 to 5. The results are also shown in Table 2. pH of each composition was measured directly from the prepared composition at room temperature (25°C) using a pH meter (manufactured by HORIBA, Ltd., F-52).

<Treatment with human hair fiber treatment agent>

1. A 25 cm long tress consisting of 0.5 g of Caucasian straight hair (untreated hair) was immersed in 40 g of the human hair fiber treatment agent in a container. The resulting container was hermetically sealed by covering the opening of the container with plastic wrap, and heated for hours described in Table 2 in an oven set to 90°C.
2. The container containing the tress was taken out of the oven and cooled to room temperature.
3. The tress was taken out of the container, rinsed under running tap water of 30°C for 30 seconds, washed with foams of shampoo for evaluation for 60 seconds, rinsed under running tap water of 30°C for 30 seconds, and slightly towel-dried. Then, the tress was dried with a warm-air dryer (manufactured by TESCOM & Co., Ltd., Nobby White NB3000). At this point in time, the tress maintained straight hair.
4. Subsequently, the tress obtained in step 3 was immersed in 40 g of the post-crosslinking agent in a container. The resulting container was hermetically sealed by covering the opening of the container with plastic wrap, and heated for 1 hour in an oven set to 90°C.
5. The container containing the tress was taken out of the oven and cooled to room temperature.
6. The tress was taken out of the container, rinsed under running tap water of 30°C for 30 seconds, washed with foams of shampoo for evaluation for 60 seconds, rinsed under running tap water of 30°C for 30 seconds, and slightly towel-dried. Then, the tress was dried with a warm-air dryer (manufactured by TESCOM & Co., Ltd., Nobby White NB3000). At this point in time, the tress maintained straight hair.

[Table 2]

| Component (% by mass) | | | Example | | | |
|---|---|---|---|---|---|---|
| | | | 1 | 6 | 7 | 8 |
| Human hair fiber treatment agent | (A) | Formaldehyde | 5 | 5 | 5 | 5 |
| | (B) | Melamine | 14 | 14 | 14 | 14 |
| | (C) | Water | Balance | Balance | Balance | Balance |
| | Others | Glutaraldehyde | - | - | - | 1.6 |
| | | Sodium hydroxide | (*) | (*) | (*) | (*) |
| | Total | | 100 | 100 | 100 | 100 |
| | pH (25°C) | | 9 | 9 | 9 | 9 |
| | Time for heating | | 1h | 2h | 2h | 2h |
| Post -crosslinking agent | (D) | Glyoxylic acid | No treated | No treated | 10 | 10- |
| | (C) | Water | | | Balance | Balance |
| | Others | Sodium hydroxide | | | (*) | (*) |
| | Total | | | | 100 | 100 |
| | pH (25°C) | | | | 2 | 2 |

(continued)

| Component (% by mass) | | Example | | | |
|---|---|---|---|---|---|
| | | 1 | 6 | 7 | 8 |
| Evaluation | Shape sustainability (value of I) | 250 | 313 | 357 | 500 |
| | Good hair surface feel | 3 | 3 | 3 | 3 |
| | Durability (Young's modulus [GPa]) | 2.14 | - | - | 2.55 |
| *: amount for pH adjustment | | | | | |

Examples 9 to 11 (Treatment with a human hair fiber treatment agent + treatment with post-crosslinking agent)

Each treatment agent shown in Table 3 was prepared and used in treatments given below to evaluate its shape sustainability, good surface feel, and durability according to the same method and criteria as in Examples 1 to 5. The results are also shown in Table 3. pH of each composition was measured directly from the prepared composition at room temperature (25°C) using a pH meter (manufactured by HORIBA, Ltd., F-52).

<Treatment with human hair fiber treatment agent>

1. A 25 cm long tress consisting of 0.5 g of Caucasian straight hair (untreated hair) was immersed in 40 g of the human hair fiber treatment agent in a container. The resulting container was hermetically sealed by covering the opening of the container with plastic wrap, and heated for 2 hours in an oven set to 90°C.
2. The container containing the tress was taken out of the oven and cooled to room temperature.
3. The tress was taken out of the container, rinsed under running tap water of 30°C for 30 seconds, washed with foams of shampoo for evaluation for 60 seconds, rinsed under running tap water of 30°C for 30 seconds, and slightly towel-dried. Then, the tress was dried with a warm-air dryer (manufactured by TESCOM & Co., Ltd., Nobby White NB3000). At this point in time, the tress maintained straight hair.
4. Subsequently, the tress obtained in step 3 was immersed in 40 g of the post-crosslinking agent in a container. The resulting container was hermetically sealed by covering the opening of the container with plastic wrap, and heated for 1 hours in an oven set to 90°C.
5. The container containing the tress was taken out of the oven and cooled to room temperature.
6. The tress was taken out of the container, rinsed under running tap water of 30°C for 30 seconds, washed with foams of shampoo for evaluation for 60 seconds, rinsed under running tap water of 30°C for 30 seconds, and slightly towel-dried. Then, the tress was dried with a warm-air dryer (manufactured by TESCOM & Co., Ltd., Nobby White NB3000). At this point in time, the tress maintained straight hair.

[Table 3]

| Component (% by mass) | | | Example | | | |
|---|---|---|---|---|---|---|
| | | | 8 | 9 | 10 | 11 |
| Human hair fiber treatment agent | (A) | Formaldehyde | 5 | 5 | 5 | 5 |
| | (B) | Melamine | 14 | 14 | 14 | 14 |
| | (C) | Water | Balance | Balance | Balance | Balance |
| | Others | Glutaraldehyde | 1.6 | 1.6 | 1.6 | 1.6 |
| | | Hydrochloric acid | (*) | (*) | (*) | (*) |
| | Total | | 100 | 100 | 100 | 100 |
| | pH (25°C) | | 9 | 9 | 9 | 9 |

(continued)

| Component (% by mass) | | | Example | | | |
|---|---|---|---|---|---|---|
| | | | 8 | 9 | 10 | 11 |
| Post -crosslinking agent | (D) | Formaldehyde | - | 4 | - | - |
| | | Glyoxylic acid | 10 | - | - | - |
| | | Glyoxal | - | - | 7.8 | - |
| | | Glutaraldehyde | - | - | - | 13.5 |
| | (C) | Water | Balance | Balance | Balance | Balance |
| | Others | Phosphoric acid | - | 10 | 10 | 10 |
| | | Sodium hydroxide | (*) | (*) | (*) | (*) |
| | Total | | 100 | 100 | 100 | 100 |
| | pH (25°) | | 2 | 2 | 2 | 2 |
| Evaluation | Shape sustainability (value of I) | | 500 | 417 | 385 | 455 |
| | Good hair surface feel | | 3 | 3 | 3 | 2 |
| | Durability (Young's modulus [GPa]) | | 2.55 | 2.29 | 2.06 | 2.01 |
| *: amount for pH adjustment | | | | | | |

Example 12 (Treatment with human hair fiber treatment agent + treatment with surface finish agent (I))

Each treatment agent shown in Table 4 was prepared and used in treatments given below to evaluate its shape sustainability, good surface feel, and durability according to the same method and criteria as in Examples 1 to 5. The results are also shown in Table 4. pH of each composition was measured directly from the prepared composition at room temperature (25°C) using a pH meter (manufactured by HORIBA, Ltd., F-52).

<Treatment with human hair fiber treatment agent>

1. A 25 cm long tress consisting of 0.5 g of Caucasian straight hair (untreated hair) was immersed in 40 g of the human hair fiber treatment agent in a container. The resulting container was hermetically sealed by covering the opening of the container with plastic wrap, and heated for 2 hours in an oven set to 90°C.
2. The container containing the tress was taken out of the oven and cooled to room temperature.
3. The tress was taken out of the container, rinsed under running tap water of 30°C for 30 seconds, washed with foams of shampoo for evaluation for 60 seconds, rinsed under running tap water of 30°C for 30 seconds, and slightly towel-dried. Then, the tress was dried with a warm-air dryer (manufactured by TESCOM & Co., Ltd., Nobby White NB3000). At this point in time, the tress maintained straight hair.
4. Subsequently, the tress obtained in step 3 was immersed in 40 g of the post-crosslinking agent in a container. The resulting container was hermetically sealed by covering the opening of the container with plastic wrap, and heated for 1 hour in an oven set to 90°C.
5. The container containing the tress was taken out of the oven and cooled to room temperature.
6. The tress was taken out of the container, rinsed under running tap water of 30°C for 30 seconds, washed with foams of shampoo for evaluation for 60 seconds, rinsed under running tap water of 30°C for 30 seconds, and slightly towel-dried. Then, the tress was dried with a warm-air dryer (manufactured by TESCOM & Co., Ltd., Nobby White NB3000). At this point in time, the tress maintained straight hair.
7. Subsequently, the tress obtained in step 3 was immersed in 40 g of the surface finish agent (I) in a container. The resulting container was hermetically sealed by covering the opening of the container with plastic wrap, and heated for 72 hours in an oven set to 40°C.
8. The container containing the tress was taken out of the oven and cooled to room temperature.
9. The tress was taken out of the container, rinsed under running tap water of 30°C for 30 seconds, washed with foams of shampoo for evaluation for 60 seconds, rinsed under running tap water of 30°C for 30 seconds, and slightly

towel-dried. Then, the tress was dried with a warm-air dryer (manufactured by TESCOM & Co., Ltd., Nobby White NB3000). At this point in time, the tress maintained straight hair.

[Table 4]

| Component (% by mass) | | | Example | |
|---|---|---|---|---|
| | | | 8 | 12 |
| Human hair fiber treatment agent | (A) | Formaldehyde | 5 | 5 |
| | (B) | Melamine | 14 | 14 |
| | (C) | Water | Balance | Balance |
| | Others | Glutaraldehyde | 16. | 1.6 |
| | | Sodium hydroxide | (**) | (**) |
| | Total | | 100 | 100 |
| | pH (25°C) | | 9 | 9 |
| Post-crosslinking agent | (C) | Water | Balance | Balance |
| | Others | Glyoxylic acid | 10 | 10 |
| | | Sodium hydroxide | (**) | (**) |
| | Total | | 100 | 100 |
| | pH (25°C) | | 2 | 2 |
| Surface finish agent (I) | (E) | Resorcinol | Not treated | 40 |
| | (C) | Water | | Balance |
| | Others | Hydrochloric acid | | (*) |
| | Total | | | 100 |
| | pH (25°C) | | | 2 |
| Evaluation | Shape sustainability (value of I) | | 500 | 500 |
| | Good hair surface feel | | 3 | 5 |
| | Durability (Young's modulus [GPa]) | | 2.55 | 2.30 |
| *: amount for pH adjustment | | | | |

Example 13 (Additional treatment with composition for cuticle removal)

Cuticle can be removed from the surface of human hair fibers by additionally performing the following steps before or after any treatment of Examples 1 to 12.

1. A 25 cm long tress consisting of 0.5 g of human hair fibers is immersed in 40 g of the composition for cuticle removal in a container and left standing for 2 hours.
2. The tress is taken out of the container, rinsed under running tap water of 30°C for 30 seconds, and slightly towel-dried. Then, the tress is dried with a warm-air dryer (manufactured by TESCOM & Co., Ltd., Nobby White NB3000).

Composition 1 for cuticle removal

| | (% by mass) |
|---|---|
| Sodium dichloroisocyanurate | 5.00 |
| Water | balance |
| pH: 5.0 | |

Composition 2 for cuticle removal

| | (% by mass) |
|---|---|
| Sodium hypochlorite | 5.00 |
| Water | balance |
| pH: 5.0 | |

Example 14 (Additional treatment with bleach composition)

Human hair fibers can be bleached by additionally performing the following steps before or after any treatment of Examples 1 to 12.

1. A 25 cm long tress consisting of 0.5 g of human hair fibers is immersed in 40 g of bleach composition 1 in a container and left standing for 2 hours.
2. The tress is taken out of the container, rinsed under running tap water of 30°C for 30 seconds, and slightly towel-dried. Then, the tress is dried with a warm-air dryer (manufactured by TESCOM & Co., Ltd., Nobby White NB3000).

Bleach composition 1

| | (% by mass) |
|---|---|
| Strong ammonia water (28% by mass) | 2.70 |
| Ammonium bicarbonate | 4.70 |
| Steartrimonium chloride (manufactured by KaoCorporation, QUARTAMIN 86W) | 8.00 |
| 1-Hydroxyethylidene-1,1-diphosphonic acid (60%by mass) (DEQUEST 2010cs) | 0.05 |
| Hydrogen peroxide water (35% by mass) | 10.90 |
| Water | balance |
| pH: 9.5 | |

Example 15 (Additional treatment with hair dye composition)

Human hair fibers can be dyed by additionally performing the following steps before or after any treatment of Examples 1 to 12.

1. A 25 cm long tress consisting of 0.5 g of human hair fibers is immersed in 40 g of any of hair dye compositions 1 to 4 in a container (the hair dye compositions 1 and 4 were each used as a 1:1 mixture of the first part and the second part) and left standing for 2 hours.
2. The tress is taken out of the container, rinsed under running tap water of 30°C for 30 seconds, and slightly towel-dried. Then, the tress is dried with a warm-air dryer (manufactured by TESCOM & Co., Ltd., Nobby White NB3000).

Hair dye composition 1

| (First part) | (% by mass) |
|---|---|
| Cetearyl alcohol | 10.8 |
| Oleth-5 | 5.0 |
| Oleic acid | 2.5 |
| Cocamide MEA | 4.6 |
| Sodium lauryl sulfate | 1.7 |
| Propylene glycol monostearate | 0.6 |
| Anhydrous sodium sulfite | 0.5 |

(continued)

| (First part) | (% by mass) |
|---|---|
| Ammonia water (28% by mass) | 6.5 |
| Toluene-2,5-diamine sulfate | 1.3 |
| Resorcinol | 0.5 |
| Metaaminophenol | 0.2 |
| 2,4-Diaminophenoxyethanol hydrochloride | 0.02 |
| Purified water | balance |

| (Second part) | (% by mass) |
|---|---|
| Hydrogen peroxide water (35% by mass) | 17.1 |
| Cetearyl alcohol | 1.7 |
| Sodium lauryl sulfate | 0.2 |
| Phosphoric acid | 0.3 |
| Salicylic acid | 0.01 |
| Purified water | balance |

Hair dye composition 2

| | (% by mass) |
|---|---|
| Acid Red 33 | 0.5 |
| Hydrochloric acid amount for pH adjustment | |
| Purified water | balance |
| pH: 4.0 | |

Hair dye composition 3

| | (% by mass) |
|---|---|
| Amodimethicone | 1.50 |
| Hydroxyethylcellulose | 1.40 |
| Fragrance | 0.30 |
| Acid Red 52 | 0.55 |
| Basic Red 51 | 0.28 |
| HC Red 3 | 0.01 |
| Water | balance |

Hair dye composition 4

| (First part) | (% by mass) |
|---|---|
| Ammonium chloride | 0.25 |
| Monoethanolamine | 0.80 |
| Fragrance | 0.30 |
| p-Toluenediamine | 0.55 |
| Resorcinol | 0.28 |

(continued)

| (First part) | (% by mass) |
|---|---|
| m-Phenylenediamine | 0.01 |
| Water pH: 6.8 | balance |

| (Second part) | (% by mass) |
|---|---|
| Hydrogen peroxide | 6.00 |
| Water pH: 3.4 | balance |

Example 16 (Treatment with surface finish agent (II))

Each surface finish agent (II) shown in Tables 5 and 6 is applied according to the bath ratio shown in the tables to the human hair fibers after any treatment of Examples 1 to 12, and the human hair fibers can be thoroughly dried using a dryer without washing off the surface finish agent, thereby imparting hydrophobicity thereto and reducing friction. In addition, these effects can be sustained over a long period after the treatment.

pH in the tables is a value measured directly from each composition without dilution or the like at room temperature (25°C) using pH meter F-52 manufactured by HORIBA, Ltd.

[Table 5]

| Content (% by mass; active component for all) | | Surface finish agent (II) | | | | | |
|---|---|---|---|---|---|---|---|
| | | 1 | 2 | 3 | 4 | 5 | 6 |
| (F) | Epoxyaminosilane copolymer (*1) | 1 | 1 | 1 | 1 | 1 | 1 |
| | Ethanol | 10 | 8 | 8 | - | - | - |
| | Benzyl alcohol | - | 2 | - | - | - | - |
| | Phenoxyethanol | - | - | 2 | - | - | - |
| | Ethyl lactate | - | - | - | 10 | - | - |
| | Diethanolamine | - | - | - | - | 10 | - |
| | Guanidine hydrochloride | - | - | - | - | - | 10 |
| | Water (*2) | 89 | 89 | 89 | 89 | 89 | 89 |
| | Lactic acid | (*3) | (*3) | (*3) | (*3) | (*3) | (*3) |
| Total amount | | 100 | 100 | 100 | 100 | 100 | 100 |
| pH | | 3.5 | 3.5 | 3.5 | 3.5 | 3.5 | 3.5 |
| Treatment bath ratio (surface finish agent (II) / human hair fibers) | | 0.5 | 0.5 | 0.5 | 0.5 | 0.5 | 0.5 |

*1: Silsoft CLX-E (manufactured by Momentive Performance Materials, Inc., polysilicone-29: 15% by mass).
*2: containing water and dipropylene glycol derived from Silsoft CLX-E
*3: amount for pH adjustment

[Table 6]

| Content (% by mass; active component for all) | | Surface finish agent (II) | | | | | | | |
|---|---|---|---|---|---|---|---|---|---|
| | | 7 | 8 | 9 | 10 | 11 | 12 | 13 | 14 |
| (F) | Epoxyaminosilane copolymer (*1) | 0.1 | 0.5 | 5 | 1 | 1 | 1 | 1 | 1 |
| | Ethanol | 10 | 10 | 10 | 5 | 7 | 20 | 93.3 | 20 |
| | Benzyl alcohol | - | - | - | - | - | - | - | 2 |
| | Phenoxyethanol | - | - | - | - | - | - | - | 2 |

(continued)

| Content (% by mass; active component for all) | | Surface finish agent (II) | | | | | | | |
|---|---|---|---|---|---|---|---|---|---|
| | | 7 | 8 | 9 | 10 | 11 | 12 | 13 | 14 |
| | Water (*2) | 89.9 | 89.5 | 85 | 94 | 92 | 79 | 5.7 | 75 |
| | Lactic acid | (*3) | (*3) | (*3) | (*3) | (*3) | (*3) | (*3) | (*3) |
| Total amount | | 100 | 100 | 100 | 100 | 100 | 100 | 100 | 100 |
| pH | | 3.5 | 3.5 | 3.5 | 3.5 | 3.5 | 3.5 | 3.5 | 3.5 |
| Treatment bath ratio (surface finish agent (II) / human hair fibers) | | 0.5 | 0.5 | 0.5 | 0.5 | 0.5 | 0.5 | 0.5 | 0.5 |
| *1: Silsoft CLX-E (manufactured by Momentive Performance Materials, Inc., polysilicone-29: 15% by mass).<br>*2: containing water and dipropylene glycol derived from Silsoft CLX-E<br>*3: amount for pH adjustment | | | | | | | | | |

**Claims**

1. A human hair fiber treatment agent for treating human hair fibers having free ends at both ends, comprising the following components (A) to (C) in the formulation thereof:

   (A): formaldehyde or hydrate thereof;
   (B): melamine derivative represented by the formula (1):

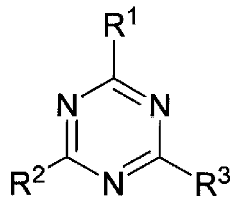

(1)

   wherein $R^1$ to $R^3$ are the same or different and each represent a hydrogen atom, a hydroxymethylamino group, a hydroxy group, a halogen atom, a phenyl group, a linear or branched alkyl group or alkenyl group having 1 or more and 6 or less carbon atoms, or a linear or branched alkoxy group or alkenyloxy group having 1 or more and 6 or less carbon atoms; and
   (C): water.

2. The human hair fiber treatment agent according to claim 1, wherein the content of the component (A) is 0.1% by mass or more and 60% by mass or less.

3. The human hair fiber treatment agent according to claim 1 or 2, wherein the content of the component (B) is 0.1% by mass or more and 60% by mass or less.

4. A method for treating human hair fibers, comprising the following step (i):

   (i) the step of immersing human hair fibers having free ends at both ends in a human hair fiber treatment agent according to any of claims 1 to 3 under heating.

5. The method for treating human hair fibers according to claim 4, wherein the bath ratio (human hair fiber treatment agent mass) / (human hair fiber mass) in immersing the human hair fibers in the human hair fiber treatment agent is 2 or more.

6. The method for treating human hair fibers according to claim 4 or 5, further comprising the following step (ii) after the step (i):
   (ii) the step of immersing the human hair fibers in a post-crosslinking agent comprising the following components

(D) and (C) :

(D): at least one formaldehyde derivative selected from the group consisting of formaldehyde, formaldehyde hydrate, glyoxylic acid, glyoxylic acid hydrate, glyoxylate, glyoxal, glyoxal hydrate, glutaraldehyde, and glutaraldehyde hydrate; and
(C): water.

**7.** The method for treating human hair fibers according to any one of claims 4 to 6, further comprising the following step (iii) after the step (i) or (ii):
(iii) the step of immersing the human hair fibers in a surface finish agent comprising the following components (E) and (C) :

(E): a resorcinol derivative represented by the formula (2):

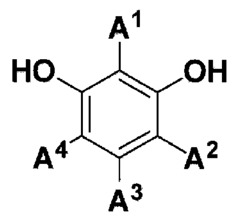

(2)

wherein
$A^1$ to $A^4$ are the same or different and each represent a hydrogen atom, a hydroxy group, a halogen atom, a carboxyl group or a salt thereof, a sulfonic acid group or a salt thereof, a linear or branched alkyl group or alkenyl group having 1 to 6 carbon atoms, or a linear or branched alkoxy group or alkenyloxy group having 1 to 6 carbon atoms; and
(C): water.

**8.** A method for producing a wig, comprising the step of treating human hair fibers for wigs by a method for treating human hair fibers according to any one of claims 4 to 7.

## INTERNATIONAL SEARCH REPORT

International application No.
PCT/JP2019/004788

**A. CLASSIFICATION OF SUBJECT MATTER**

Int. Cl. A41G3/00(2006.01)i, D06M13/12(2006.01)i, D06M13/152(2006.01)i, D06M15/41(2006.01)i, D06M15/423(2006.01)i

According to International Patent Classification (IPC) or to both national classification and IPC

**B. FIELDS SEARCHED**

Minimum documentation searched (classification system followed by classification symbols)
Int. Cl. A41G3/00, D06M13/12, D06M13/152, D06M15/41, D06M15/423

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 9-59873 A (TOYOBO CO., LTD.) 04 March 1997, paragraphs [0004]-[0015] (Family: none) | 1-8 |
| Y | JP 2006-16310 A (KAO CORP.) 19 January 2006, paragraphs [0018], [0027]-[0029] (Family: none) | 1-8 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13.03.2019 | 26.03.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005037000 A
- JP 2007177370 A